# EUROPÄISCHE PATENTANMELDUNG

(11) **EP 0 761 222 A1**
(43) Veröffentlichungstag der Anmeldung: **12.03.1997**
(21) Anmeldenummer: 96112848.5
(22) Anmeldetag: 09.08.1996
(51) Int. Cl.: A61K 31/505

(54) **Verwendung von Orotsäure zur Herstellung eines Arzneimittels zur Verbesserung der Mikrozirkulation**

(30) Priorität: 17.08.1995 DE 19530301
(71) Anmelder: WÖRWAG PHARMA GmbH, 71034 Böblingen (DE)
(72) Erfinder: Wörwag, Fritz, Dr., 70192 Stuttgart (DE)
(74) Vertreter: Weller, Wolfgang, Dr.rer.nat.

(57) **Zusammenfassung**

Durch histologische sowie koronarangiographische Untersuchungen konnte nachgewiesen werden, daß durch Orotsäure und Magnesiumorotat Verschlüsse der kleinen und kleinsten Blutgefäße verhindert bzw. in ihrer Ausprägung reduziert werden konnten.

Da Orotsäure und Magnesiumorotat direkt an den Auslösemechanismen für Störungen der Mikrozirkulation angreifen, nämlich eine antiarteriosklerotische und blutdrucksenkende Wirkung zeigen, und zugleich die Fließeigenschaften des Blutes als solchem verbessern, kann durch diese Therapeutika die Mikrozirkulation des Gewebes erheblich verbessert werden und Mikroangiopathien präventiv oder therapeutisch behandelt werden.

## Beschreibung

Die Erfindung betrifft ein Produkt für die Verbesserung der Mikrozirkulation.

Unter dem Begriff Mikrozirkulation wird die Blutzirkulation in den Blutkapillaren verstanden, also in den einen extrem kleinen Durchmesser aufweisenden Blutgefäßen.

Wichtig für die Mikrozirkulation ist die sogenannte Endstrombahn, also der aus Arteriolen, Kapillaren und postkapillaren Venen bestehende Abschnitt des Gefäßsystemes. Nach hämodynamischer Definition handelt es sich um den neutralen Bereich des terminalen Kapillarbettes zwischen artiellem Influx und venösem Efflux, also dem Wendepunkt des Kreislaufes. In diesem submakroskopischen Gefäßabschnitt spielt sich die gesamte nutritive Blutversorgung ab, d.h. dort findet der Stoff- und Gasaustausch zwischen Blut und Gewebe statt.

Die Funktionen dieser Endstrombahn besteht in der Versorgung aller peripheren und zentralen Zellen mit Sauerstoff und Nährstoffen, in der Aufrechterhaltung des richtigen thermalen Milieus und des richtigen Ionenmilieus.

Eine Störung dieser Mikrozirkulation kommt u.a. bei arteriellen Verschlußkrankheiten, im Schock, bei Verbrennungen und anderen Erkrankungen mit rheologischen Störungen vor. Diese Störungen manifestieren sich in einer Mikrothrombosierung, Steigerung der Kapillarpermeabilität, etc. und können damit zu Durchblutungsstörungen des Gewebes führen, das durch diese Gefäße mit Nährstoffen und Sauerstoff versorgt wird.

Eine mit Störungen der Mikrozirkulation im Zusammenhang stehende Gefäßkrankheit ist die Mikroangiopathie, ein durch Stenosierung und Thrombosierung kleiner und kleinster arterieller Gefäße bedingtes Krankheitsbild. Zu den typischen Formen der Mikroangiopathie gehört die diabetische Mikroangiopathie, ein typisches Spätsyndrom des Diabetes mellitus, sowie die Mikroangiopathie bei Sklerodermie, also bei arteriellen Verschlüssen mit entsprechenden Durchblutungsstörungen.

Bisher wurden Störungen der Mikrozirkulation bzw. Angiopathien mit durchblutungsfördernden Medikamenten, Lipidsenkern und Kardiaka behandelt. Es wurden also im wesentlichen die verschiedenen Symptome behandelt, die mit diesem Krankheitsbild einhergehen, nämlich die Arteriosklerose, die Fettstoffwechselstörung sowie Herzerkrankungen.

Hiervon ausgehend ist es Aufgabe der vorliegenden Erfindung, ein Produkt zur Verbesserung der Mikrozirkulation bereitzustellen.

Erfindungsgemäß wird diese Aufgabe dadurch gelöst, daß Orotsäure zur Verbesserung der Mikrozirkulation verwendet wird.

Orotsäure wird bisher in Salzform als sogenannter Mineral- oder Elektrolytschlepper eingesetzt und dient dazu, bestimmte Kationen in die Zelle zu transportieren. Aus der DE-OS-24 10 181 ist es beispielsweise bekannt, Lithiumorotat einzusetzen, um Natrium, das bei der Regulierung des Wasserhaushaltes der Zelle eine Rolle spielt, in der Zelle durch Lithium zu ersetzen. Lithium wird hier in Form des Salzes der Orotsäure verwendet, da dieses den Transport des Lithiums in die Zelle bewirken kann.

Durch die Deplazierung des Natriums durch Lithium wird die Menge an gespeichertem Wasser reduziert. Dieser Effekt wird zur Therapie von Hypertonie, Arteriosklerose, Gefäß- und Gewebserkrankungen insbesondere im zerebralen Bereich sowie zur Dauer- und Schutzbehandlung von Migräne ausgenutzt.

Überraschenderweise konnte jetzt gefunden werden, daß die Orotsäure selbst im Zusammenhang mit der Verbesserung der Mikrozirkulation präventiv und therapeutisch wirksam eingesetzt werden kann.

Die Orotsäure kann dabei direkt oder in Form eines ihrer Salze verwendet werden, wobei vorzugsweise Magnesiumorotat eingesetzt wird.

In Studien konnte gezeigt werden, daß Orotsäure nicht nur arteriosklerotischen Gefäßveränderungen unter experimentellem Cholesterinstreß entgegenwirkt sondern, was für die Verbesserung der Mikrozirkulation entscheidender ist, daß die Fließeigenschaften des Blutes verbessert werden, dieses also besser durch die Kapillaren strömt.

Dabei zeigte sich, daß die Wirkung der reinen Orotsäure deutlich besser war als die von MgCl₂, so daß der Wirkeffekt bei Magnesiumorotat nicht primär auf Magnesium, sondern auf die Orotsäure selbst zurückzuführen ist.

Weiterhin wurde in Tierexperimenten gezeigt, daß Orotsäure blutdrucksenkend wirkt.

Durch histologische sowie koronarangiographische Untersuchungen konnte nachgewiesen werden, daß durch Orotsäure und Magnesiumorotat Verschlüsse der kleinen und kleinsten Blutgefäße verhindert bzw. in ihrer Ausprägung reduziert werden konnten.

Da Orotsäure und Magnesiumorotat direkt an den Auslösemechanismen für Störungen der Mikrozirkulation angreifen, nämlich eine antiarteriosklerotische und blutdrucksenkende Wirkung zeigen, und zugleich die Fließeigenschaften des Blutes als solchem verbessern, kann durch diese Therapeutika die Mikrozirkulation des Gewebes erheblich verbessert werden.

Dieser Effekt ist überraschend und war in diese Deutlichkeit so nicht zu erwarten.

Somit können Orotsäure und Magnesiumorotat auch zur Prävention und Therapie von Mikroangiopathien verwendet werden, da die mit diesem Krankheitsbild zusammenhängenden Störungen der Mikrozirkulation durch die genannten Therapeutika verringert werden.

Schließlich konnte in einer Untersuchung an Triathleten gezeigt werden, daß nach einer Magnesiumorotat-Supplementierung das Insulin besser genutzt werden kann, was sich durch eine bessere Glukoseverwertung zeigt. Das bedeutet jedoch, daß die Verabreichung von Orotsäure oder Magnesiumorotat auch zu einer Verbesserung des Diabetes mellitus führt, so daß auch dieser Auslöser von Mikroangiopathien ursächlich therapiert werden kann.

In den bereits erwähnten Experimenten konnte weiter gezeigt werden, daß die Verwendung von Magnesiumorotat gegenüber der Verwendung von reiner Orotsäure eine noch größere präventive und therapeutische Wirkung zeigt.

In einem Ausführungsbeispiel ist es dann bevorzugt, wenn eine Mischung aus Orotsäure und einer Magnesiumverbindung verwendet wird.

Hier ist von Vorteil, daß ein synergistischer Effekt auftritt, zum einen wird die Wirkung der Orotsäure ausgenutzt, wobei zusätzlich eine Magnesiumverbindung eingesetzt wird, um auch deren Wirkung mit zu verwenden.

Die Mischung besteht dabei vorzugsweise aus einem Salz der Orotsäure und einer Magnesiumverbindung, wobei auch eine Mischung aus Orotsäure und Magnesiumorotat verwendet werden kann.

Auch hier ist von Vorteil, daß neben der reinen Orotsäure Magnesium in dem herzustellenden Arzneimittel/Präparat/Produkt zu finden ist.

Das aus Orotsäure und/oder ihren Salzen hergestellte Arzneimittel/Produkt kann dabei in allen Darreichungsformen auftreten.

Die Erfindung wird nachfolgend anhand einiger Studien und im Zusammenhang mit den Figuren näher beschrieben und erläutert.

Es zeigen:
- Fig. 1: Indizes arteriosklerotischer Gefäßveränderungen unter experimentellem "Cholesterinstreß" bei Kaninchen; und
- Fig. 2: die Reduktion der in Wistar-Ratten experimentell induzierten, malignen Blutdrucksteigerung durch Magnesiumorotat.

### Beispiel 1: Einfluß von Magnesiumorotat auf die Fließeigenschaft von menschlichem Blut

10 Patienten mit Durchblutungsstörungen wurden 4 g Magnesiumorotat zusammen mit ca. 150 ml Wasser verabreicht. Die Blutentnahme erfolgte vor und im Abstand von 15, 30, 60, 120 und 180 Minuten nach der Tabletteneinnahme. Direkt vor jeder Blutentnahme wurden Blutdruck, Puls, der transkutane Sauerstoffdruck, der transkutane Kohlendioxiddruck und die Heizleistungsabweichung gemessen. Es wurden folgende Blutparameter untersucht:
- Hämatokrit
- BSC
- Plasmaviskosität
- Erythrozytenaggregation
- Erythrozytenfiltrabilität.

Es wurde eine Senkung des systemischen Druckes, insbesondere des systolischen Druckes, bis zum Zeitpunkt 60 Minuten nach Tablettenvergabe festgestellt. Die relative Plasmaviskosität erreicht 60 Minuten nach Tabletteneinnahme ihr Minimum mit anschließender Tendenz zur Normalisierung. In ähnlicher Weise verhält sich die Blutviskosität, die 30 Minuten nach Tabletteneinnahme ein Minimum aufweist. In Parallelität zum Verhalten der Blut- und Plasmaviskosität ist der Hämatokritwert zu sehen, auch hier gibt es eine temporäre Abnahme der Werte mit Tendenz zum Wiederanstieg zum Versuchsende.

Der Abfall der systolischen Blutdruckwerte ohne Erhöhung der Pulsfrequenz und die zeitgleiche Senkung der Blut- und Plasmaviskosität einschließlich der Hämatokritwerte lassen sich über den Mechanismus einer sogenannten inneren Hämodilution erklären.

Besonders interessant sind die Veränderungen der transkutanen Sauerstoffdruckwerte, die im Bereich des Vorfußes abgenommen werden. Bei diesen Patienten mit einer chronisch-arteriellen Verschlußkrankheit sind die tcpO₂-Werte bekannterweise nicht sonderlich erniedrigt. Die Ergebnisse der pO₂-Werte zeigen doch, daß es zu einem Anstieg der pO₂-Werte kommt, die zu dem Zeitpunkt 15 und 30 Minuten statistisch gesichert sind. Dieser Anstieg der tcpO₂-Werte überrascht und beruht auf den verbesserten Fließeigenschaften des Blutes.

Überraschend wurde festgestellt, daß es zu keinem Zeitpunkt nach der oralen Gabe von Magnesiumorotat zu statistisch signifikanten Veränderungen der Erythrozytenfiltrationswerte kam.

Zusammenfassend ist zu sagen, daß nach akuter oraler Gabe von 4 g Magnesiumorotat bei Patienten mit arteriellen Verschlußkrankheiten und damit verbundenen Mikrozirkulationsstörungen neben einer temporären Senkung der systemischen Blutdruckwerte auch die Blut- und Plasmaviskosität signifikant vermindert wird.

Dies führt zu einer erheblich verbesserten Mikrozirkulation, die durch Messung des Kapillarflusses an dem Nagelbett festgestellt wurde.

### Beispiel 2: Untersuchung der Wirkung von Orotsäure und Magnesiumorotat in einem Arteriosklerose-Modell

In einer Versuchsreihe mit einem Arteriosklerose-Modell wurden Kaninchen über 112 Tage einer zweiprozentigen Cholesterindiät ausgesetzt und mit äquimolaren Mengen von` Magnesiumchlorid, Orotsäure und Magnesiumorotat bzw. in der Kontrollgruppe überhaupt nicht weiter behandelt. Zusätzlich zu histologischen Untersuchungen der Koronaarterien sowie der Aorta femoralis und renalis wurde der Zustand der Aorta an jeweils mehreren Stellen auch planimetrisch-photographisch dokumentiert. Mittels einer computergestützten, digitalen Methode der Oberflächenmessung ("density slice"-Methode) erhält man hierbei dreidimensionale Bilder, mit deren Hilfe auch eine Quantifizierung der cholesterininduzierten Lumeneinengungen möglich ist.

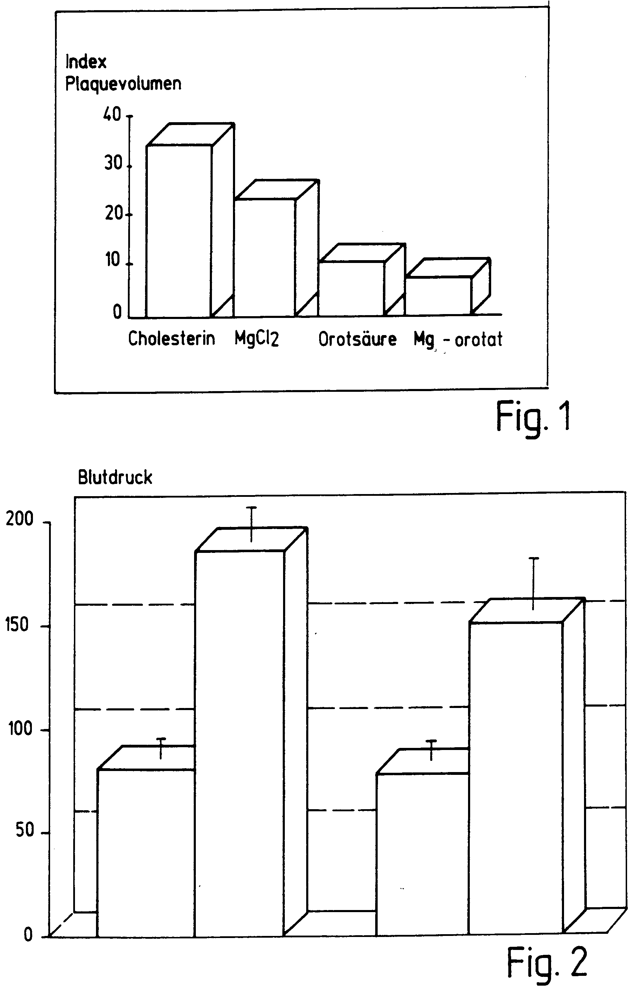

In Fig. 1 ist das Ergebnis dieser Untersuchung dargestellt. Es ist zu erkennen, daß die Gefäßverengungen in der unbehandelten Kontrollgruppe mit einem Index von 34,36 am ausgeprägtesten waren. Die Orotsäure wirkt mit einem Index von 9,77 deutlich besser den arteriosklerotischen Läsionen entgegen als Magnesiumchlorid (Index 22,63).

Als bester Schutz erwies sich jedoch Magnesiumorotat mit einem Index von 7,28.

Diese quantifizierten Ergebnisse wurden durch die licht- bzw. elektronenmikroskopischen Befunde sowie durch enzymhistochemische Untersuchungen bestätigt. Demnach verhütet Magnesiumorotat auch in den Koronarien die Genese sklerotischer Läsionen am besten. Die lumeneinengende, durch die Cholesterinfütterung induzierte Proliferation von Schaumzellen war unter dieser Medikation weitaus am geringsten ausgeprägt.

Aus dieser Untersuchung ergibt sich, daß Orotsäure selbst arteriosklerotische Gefäßveränderungen entscheidend verhütet und daß diese Wirkung durch Zugabe von Magnesium weiter gesteigert werden kann.

### Beispiel 3: Koronarangiographische Untersuchungen zu Beispiel 2

Zusätzlich zu den planimetrisch-photographischen Untersuchungen wurde bei der Versuchsreihe aus Beispiel 2 auch eine koronarangiographische Untersuchung vorgenommen.

Es zeigte sich, daß der experimentell hervorgerufene Cholesterinstreß in der unbehandelten Kontrollgruppe zu Mikroangiopathien in Koronarien führt.

In der Verumgruppe, die mit Orotsäure bzw. Magnesiumorotat behandelt wurde, ergab sich eine präventive und therapeutische Wirkung bei Mikroangiopathien von kleinen und kleinsten Blutgefäßen am Herzen.

### Beispiel 4: Untersuchung der Wirkung von Magnesiumorotat im Hypertonie-Tiermodell

In den Hypertonie-Tiermodellen nach Rojo-Ortega-Genest (ROG-Modell) und Lörincz-Goracz (LG-Modell) wird bei Wistar-Ratten die Aorta zwischen den beiden Renalarterien unterbunden bzw. die Niere in eine Gummimanschette fest eingepreßt, was einen malignen Hypertonus hervorruft, also eine Hypertonie mit konstanter Erhöhung des arteriellen diastolischen Blutdruckes auf > 120 mmHg.

Unter täglicher Zufuhr von 300 mg Magnesiumorotat waren weit weniger periarterielle Zellinfiltrationen und Herzmuskelzellausfälle zu beobachten als ohne Behandlung.

Das Ergebnis dieser Untersuchung ist in Fig. 2 dargestellt, wobei die dunklen Säulen jeweils den Beginn der ROG-Hypertonie anzeigen, während die hellen Säulen das Ende der ROG-Hypertonie darstellen. Links in Fig. 2 ist die ROG-Hypertonie ohne Behandlung gezeigt, während die beiden rechten Säulen den Verlauf der ROG-Hypertonie unter Zugabe von täglich 300 mg Magnesiumorotat wiedergibt.

Diese Therapie reduzierte die experimentiell induzierte, maligne Blutdrucksteigerung per se. Der Wirkeffekt des Magnesiumorotats ergibt sich dabei aus der Differenz zwischen den beiden hellen Säulen. Der Blutdruck betrug im ROG-Modell 152 mmHg systolisch gegenüber 186 mmHg bei unbehandelten Kontrolltieren nach Ausgangswerten von 79 bzw. 81 mmHg, war also bei den behandelten Tieren deutlich geringer ausgeprägt.

### Beispiel 5: Untersuchung der Wirkung von Magnesiumorotat auf den Energiestoffwechsel von Triathleten

In einer doppelblinden, randomisierten und placebokontrollierten Studie wurden 23 Triathleten einem 500 m Schwimm-, 20 km Fahrrad- und 5.000 m Lauftest nach einer vierwöchigen Magnesiumorotat-Supplementierung von 17 mmol/Tag unterworfen. Die Tests wurden ohne Pausen direkt hintereinander absolviert. Vor dem Test, zwischen den Disziplinen und am Ende wurde den Probanden Blut entnommen und auf Parameter des Energiestoffwechsels untersucht.

Vor dem Triathlon-Test waren die Glukosekonzentrationen beider Gruppen vergleichbar. Während des Tests stieg die Glukosekonzentration der Kontrollgruppe um 187 ± 34 %, die der Verumgruppe signifikant niedriger um 118 ± 45 % an. Gleichzeitig erhöhte sich die Insulinkonzentration der Kontrollgruppe durch den Streß auf 139 ± 101 %, in der Verumgruppe fiel die Insulinkonzentration im Vergleich zur Kontrolle dagegen signifikant auf 65 ± 43 % der Anfangswerte ab.

Diese streßabhängigen Veränderungen des Energiestoffwechsels deuten darauf hin, daß nach einer Magnesiumorotat-Supplementierung die Glukose aufgrund der besseren Insulinnutzung auch bei Diabetikern besser verwertet wird.

Die obigen Beispiele zeigen, daß Orotsäure und Magnesiumorotat direkt an den Auslösemechanismen für Störungen der Mikrozirkulation angreifen, sie zeigen nämlich eine antiarteriosklerotische und blutdrucksenkende Wirkung und führen aufgrund der Verbesserung der Fließeigenschaften des Blutes auf direkte Weise auch dazu, daß Mikroangiopathien ursächlich therapiert werden können. Orotsäure und Magnesiumorotat führen bei Diabetikern ferner auf indirekte Weise über eine Verbesserung des Diabetes mellitus ebenfalls zu einer Therapierung von Mikroangiopathien.

## Patentansprüche

1. Verwendung von Orotsäure zur Herstellung eines Arzneimittels zur prophylaktischen und therapeutischen Verbesserung der Mikrozirkulation.

2. Verwendung von Orotsäure zur Herstellung eines Arzneimittels zur Prävention und Therapie von Mikroangiopathien.

3. Verwendung nach Anspruch 1 oder Anspruch 2, dadurch gekennzeichnet, daß Störungen der Mikrozirkulation verringert werden.

4. Verwendung nach einem der Ansprüche 1 - 3, dadurch gekennzeichnet, daß ein Salz der Orotsäure verwendet wird.

5. Verwendung nach Anspruch 4, dadurch gekennzeichnet, daß Magnesiumorotat verwendet wird.

6. Verwendung nach einem der Ansprüche 1 - 5, dadurch gekennzeichnet, daß eine Mischung aus Orotsäure und einer Magnesiumverbindung verwendet wird.

7. Verwendung nach Anspruch 6, dadurch gekennzeichnet, daß eine Mischung aus einem Salz der Orotsäure und einer Magnesiumverbindung wird.

8. Verwendung nach Anspruch 6 oder Anspruch 7, dadurch gekennzeichnet, daß eine Mischung aus Orotsäure und Magnesiumorotat verwendet wird.
